Europäisches Patentamt

European Patent Office

Office européen des brevets

(11) Publication number: 0 330 897
A2

# (12) EUROPEAN PATENT APPLICATION

(21) Application number: 89102456.4

(51) Int. Cl.⁴: C12Q 1/68 , G01N 27/26

(22) Date of filing: 13.02.89

(30) Priority: 04.03.88 JP 49660/88

(43) Date of publication of application:
06.09.89 Bulletin 89/36

(84) Designated Contracting States:
DE GB

(71) Applicant: HITACHI, LTD.
6, Kanda Surugadai 4-chome
Chiyoda-ku Tokyo 101(JP)

(72) Inventor: Simada, Tamotsu
201, 948-3, Fukujima-cho
Akishima-shi Tokyo(JP)
Inventor: Nagai, Keiichi
9-204, 3-44-14, Sakuragaoka
Higashiyamato-shi Tokyo(JP)
Inventor: Tokita, Jiro
3-32-7, Nishifu-cho
Fuchu-shi Tokyo(JP)
Inventor: Nakano, Ryusei
Hyakuta-ryo, 428-1, Higashi-toyoi
Kudamatsu-shi Yamaguchi(JP)
Inventor: Sumitani, Tomoaki
3-13-6-305, Touwa
Adachi-ku Tokyo(JP)
Inventor: Watanabe, Kenichi
Hyakuta-ryo, 428-1 Higashi-toyoi
Kudamatsu-shi Yamaguchi(JP)

(74) Representative: Ebbinghaus, Dieter et al
Patentanwälte v. Füner, Ebbinghaus, Finck
Mariahilfplatz 2 & 3
D-8000 München 90(DE)

(54) Apparatus for determining base sequence of nucleic acid.

(57) During the electrophoresis analysis of one or more sample sets, a controller (31) may be used to control the appartus to permit the adding of a second sample, without disrupting the analysis of the first sample, with such adding being capable of being performed at any time during the analysis period of any sample within the entire electrophoresis apparatus. That is, a plurality of analysis periods may overlap each other with respect to different samples applied at different times. Decoding of the base sequence is performed on a real time basis together with the detecting of radiation and the processing of the detected information.

FIG. 1

# APPARATUS FOR DETERMINING BASE SEQUENCE OF NUCLEIC ACID

## BACKGROUND OF THE INVENTION

The present invention relates to an apparatus and method for determining the base sequence of a nucleic acid (DNA).

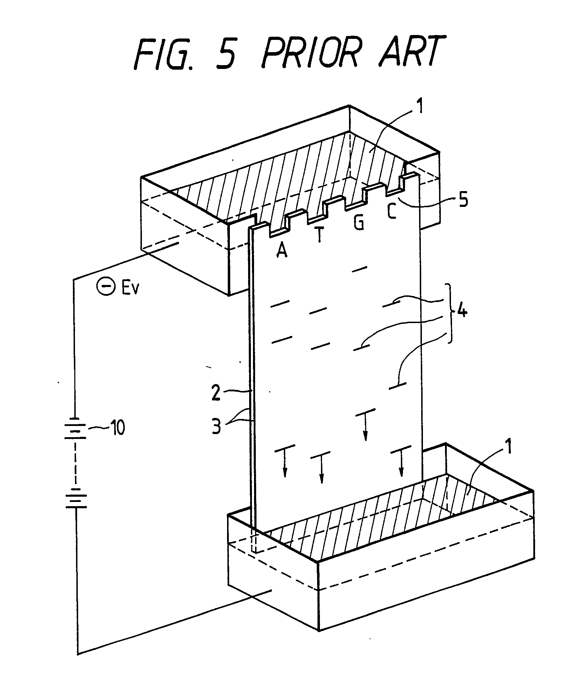

The base sequences of DNA have theretofore been determined by depending upon, for example, the Maxam Gilbert method. According to this method, a nucleic acid is labeled with a radioisotope and chemically fragmented. The fragments of the nucleic acid having different lengths are arranged by the electrophoretic method in order of their molecular weights in supporting gel sandwiched between glass plates. The supporting gels are then pealed off of the glass plates and an autoradiogram is taken to detect the electrophoretic bands that contain fragments of a radioactive nucleic acid, thereby determining the sequence of the bases of the nucleic acid. The method of determining the base sequence of fragments of nucleic acid according to the prior art will now be described with reference to Fig. 5.

Fig. 5 is a perspective view illustrating the structure of a conventional electrophoretic apparatus for fragments of a nucleic acid. The device comprises a gel 2 for electrophoretic separation of nucleic acid fragments sandwiched in between two glass plates 3. An electrode solution vessel 1, is provided at both the top and bottom of the gel to respectively immerse the opposite ends of gel for electrophoretic separation. A high dc voltage is applied through electrodes to the opposite ends of the gel by a power source 10. A sample of nucleic acid that has been fragmented and labeled with a radioisotope, for example $^{32}$P, is supplied to the wells 5 on the negative electrode side of the gel 2 for electrophoretic separation. The fragments are moved under the application of the voltage Ev of about 50 volts per centimeter along the length of the gel. The nucleic acid fragments have the same molecular weight move from the negative electrode towards the positive electrode while forming electrophoretic bands 4 with a mobility nearly in inverse proportion to the logarithm of the molecular weight. The movement is stopped after a suitable period of time has passed, the analyzing period, and an X-ray film is brought into intimate contact with the gel for exposure to the radiation from the isotopes. Appearing migration bands as an exposure pattern corresponding to the fragments of the nucleic acid is then obtained on the X-ray film. Based on this pattern, the nucleic acid fragments are read starting from the shorter ones to determine the sequence of bases of the nucleic acid.

However, this method takes one entire day for the exposure of the X-ray film, and it can determine only about 200 to 300 bases.

A second type of prior art that solves the above mentioned problem relating to the slow exposure of the X-ray film and which is capable of easily determining the sequence of the bases within a short period of time, has been disclosed in Japanese Patent Laid-Open No. 161559/1985. According to this apparatus, a detector is provided on the path of the segments of the nucleic acid in the gel for electrophoretic separation. The movement is continued to detect and read radioactive rays from the radioisotope labeling of the nucleic acid fragments that pass in front of the detector. With the label of the nucleic acid fragment composed of a fluorescent material, fluorescence may be detected using a light detector. The present invention is applicable to either light radiation detecting or nuclear radiation detecting, corresponding respectively to either fluorescent labeling or radioisotope labeling.

## SUMMARY OF THE INVENTION

According to the above mentioned prior art, consideration has not been given to analyzing a plurality of samples and a user has a problem of using the apparatus when a plurality of samples are to be analyzed. The samples must have all been prepared just before analyzing the samples.

It is an object of the present invention to provide an apparatus for determining the base sequence of nucleic acid. It is capable of continuously analyzing the samples that are prepared at any time. It is a further object to provide for real time decoding of the base sequence of nucleic acid during analyzing involving detecting rays from the gel during electrophoretic processing.

The gel for electrophoresis has, for example, twenty or more wells so that a number of samples can be simultaneously analyzed. As is well known, the nucleic acid is constituted by four kinds of bases, that is adenine (A), guanine (G), cytosine (C) and thymine(T). The fragmentation may be by different fragmenting methods to obtain the four kinds of sample fragments at proportions of their respective bases. These four kinds of fragments will constitute one set of a sample. The term "set" is also intended to include additional kinds, because depending upon the cases, a reference may be added and therefore the set may constitute five kinds, for example.

Using conventional apparatus, all of the sets must be prepared beforehand and placed in the wells, so that electrophoresis for all samples may be started simultaneously and not thereafter interrupted.

Using the apparatus of the present invention, electrophoresis of one sample set may be well advanced in its analyzing period, and a separate set may be added to the process or apparatus.

When a set is to be added, while the apparatus of the present invention is in operation, the power source for the electrophoresis is turned off. In this case, the sample being measured, discontinues the electrophoresis, and the operation of, for example, a pulse counter of the signal processing unit is stopped in synchronism with interrupting the power source for electrophoresis. The new sample is then supplied, sample loading, which takes about three minutes. The new set of sample has been previously prepared, which takes about one hour, during the electrophoresis of the first set. After the second set is loaded, the power source for electrophoresis is turned on and the pulse counter of the signal processing apparatus is operated again to restart the measurement. Previous measure values or information processed therefrom is stored during the hold period while loading the second sample, and this information is then used with respect to the first set upon restarting the apparatus. The above mentioned operation makes it possible to flawlessly process the signals of the sample that is being measured on a continuous basis, despite adding additional samples during the analyzing process or period of the first sample set.

## BRIEF DESCRIPTION OF THE DRAWING

Further objects, features and advantages of the present invention will appear from the following detailed description of a preferred embodiment shown in the drawing, wherein:

Fig. 1 is a diagram illustrating the structure of the system according to the present invention;

Fig. 2 is a diagram of the system according to Fig. 1, in more detail;

Fig. 3 is a flow chart of the method of operating the apparatus shown in Figs. 1 and 2;

Fig. 4 is a diagram showing how the apparatus of Fig. 1 and 2 and the method of Fig. 3 may be duplicated under the control of a single computer to be part of a larger system; and

Fig. 5 is a perspective view of a conventional apparatus for electrophoresis.

## DETAILED DESCRIPTION OF THE DRAWING

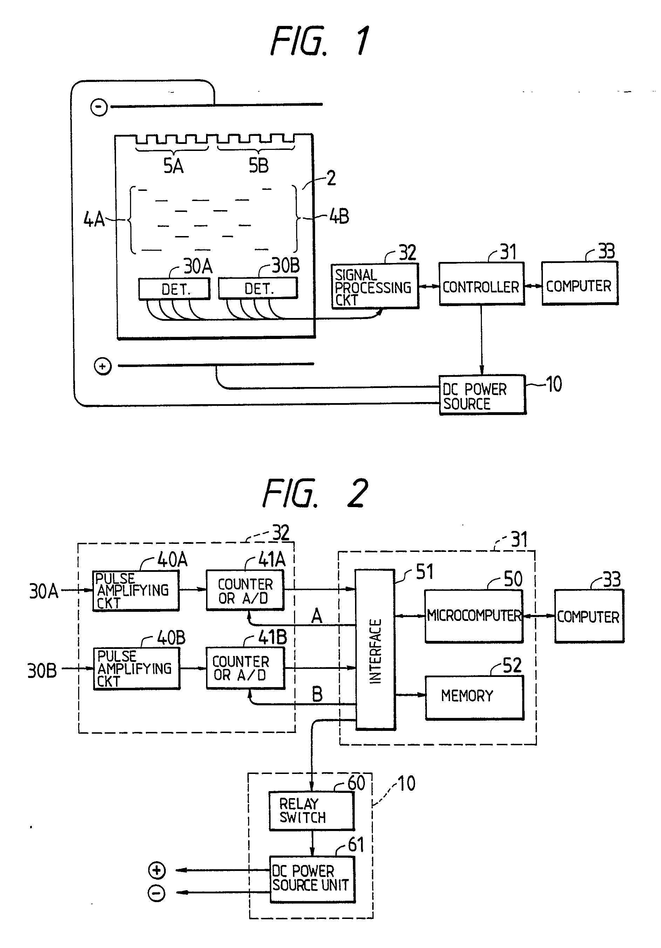

As shown in Fig. 1, a sample A is supplied to wells 5A (4 wells being shown respectively for the fragments A, C, G, and T) in electrophoretic gel 2. The power source 10 for applying the voltage across the electrophoretic gel is turned on under the control of a main computer 33 and a controller 31, in order to provide the electrophoresis of the sample A. In order to detect Beta-rays from the DNA fragments 4A of the nucleic acid, the detector 30A and the signal processing circuit 32 are operated. The detector 30A is provided with a number of separate detectors corresponding in number to the number of wells 5A. The data obtained from the signal processing circuit 32 is passed through the controller 31 to the computer 33 to automatically determine the base sequence of nucleic acid on a real time basis with the detection.

Next, when a sample B is to be added to the apparatus, the dc power source 10 is turned off. Therefore, electrophoresis of the sample A being measured is interrupted. The signal processing circuit stops its operation. However, the controller 31 or the computer 33 has stored in the temporary memory the information relating to the previous detection and previous signal processing, which is held in memory during the time that the power source 10 is turned off. At the same time with turning off the power source, the detecting and signal processing is interrupted. The control operation is carried out by the computer 33 and the controller 31. After the sample B has been added to the apparatus, particularly by being provided to the wells 5B, the computer 33 and the controller 31 will again turn on the dc power source 10 and the signal processing circuit 32 simultaneously, whereby the samples A and B can be continuously analyzed.

Fig. 2 specifically illustrates the structure of an apparatus for controlling the dc power source and the signal processing circuit using a computer and a controller. First, the signal processing circuit 32 contains pulse amplifier circuits 40A, 40B and counters 41A, 41B to count signals from the detectors 30A and 30B. Each of the amplifier circuits 40A, 40B and counters 41A, 41B separately process and output the separate signals from the plurality of detectors 30A, 30B that correspond in number to the number of wells (eight being shown for 5A, 5B). The DC power source 10 is constituted by a relay switch 60 and a DC power source unit 61. These are controlled by the controller 31, which is constituted by a microcomputer 50, interface 51 and a memory 52. For example, when a sample B is to be added while the sample A is being measured by the detector 30A, control signals from the computer 33 are decoded by a program of the microcomputer 50 to carry out the control operation as described below.

First the output voltage of dc power source unit 61 is turned off by the relay switch 60 via the interface 51. In the next step of the program of the microcomputer 50, the data of the counter 41A is stored in the memory 52 to stop the operation of the counter (control signal A). Next, the user loads the sample B, which may take about three minutes, and then the user gives the instruction to the computer 33 to resume the measurement. During the loading of the new set, the first set is holding, that is there is interruption or disablement of any portion of the analyzing of the first set sufficient to prevent errors when the power is again turned on for electrophoresis, for example interrupting one or more of detecting, processing and storing. The microcomputer 50, on a real time basis, decodes the control signals of the computer. The microcomputer 50 turns the dc power source unit 61 on using the relay 60 to resume the electrophoresis. The microcomputer 50 operates counter 41A again and actuates the counter 41B, whereby measurement of the sample A is continued and measurement of the sample B is carried out also. In the foregoing description, samples have been measured in two sets. However, the samples can be treated in the same manner even when they are to be measured in larger numbers.

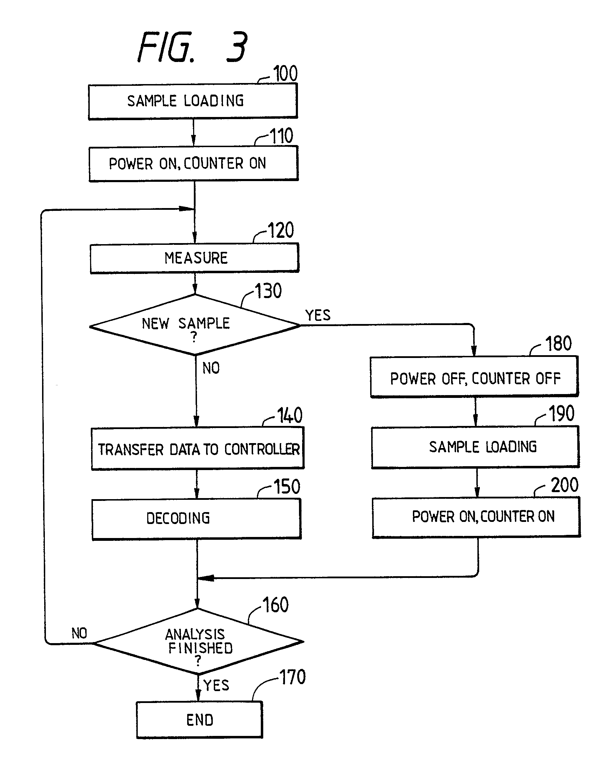

As shown in the flow chart of Fig. 3, a sample A is loaded according to step 100, for the method as performed with respect to the apparatus of Figs. 1 and 2. In step 110, the power source for electrophoresis is turned on and the pulse counter is turned on. According to step 120, the detector measures radiation from the sample and produces electrical detection signals accordingly. According to step 130, it is determined whether a new sample is to be added or not. If the answer to this determination is no, the data obtained from the measurement at 120 is transferred to the controller in step 140 (step 140 could be between steps 120 and 130). On a real time basis, step 150 decodes the base sequence of the nucleic acid according to processing of the signals obtained from the measuring step 120. At step 160, a determination is made as to whether or not the analyzing period has ended. The analyzing period may be set automatically by the program within the computer or entered by a user. If the analyzing period has not ended, which may be five to eight hours, the "no" answer returns the process to a point between steps 110 and 120, to again follow the steps beginning with step 120 as mentioned above. If a "yes" answer is obtained with step 160, the process proceeds to step 170, and the process, for at least sample A, is ended. Of course, with additional samples, steps 120, 140 and 150 would be repeated for each separate sample and deactivated when their respective analyzing periods end, and step 160 would only answer "yes" if all the analyzing periods have ended. More specifically, step 160, upon determining if a specific analyzing period has ended, would disable the steps 120, 140, 150 associated with that period and still answer "no" if any analyzing period remained unfinished.

If the answer to step 130 is "yes", the program proceeds to step 180, where the power for electrophoresis is turned off, simultaneously the power to the counter is turned off. With respect to turning off the power to the counter, this is meant to include alternately turning off power to the measurement device, turning off power to the signal processing, and the like. Further, step 150 of decoding, if used, may be placed before step 130; also step 150 may be conducted only once for each plurality of steps of detecting so long as it is conducted between two steps of detecting to be on a real time basis. From step 180, the process proceeds to step 190, which is a hold period of time during which the next sample is added. This hold period of time is ended by the user to proceed to step 200, where the power for electrophoresis is turned on simultaneously with power being turned on for the counter. The process is then returned to step 160. Step 140 is intended to include storing the data of measurement or storing of process signals corresponding to the same.

Therefore, operation of the pulse counter of the signal processing device is interrupted in synchronism with turning off the power for electrophoresis. After the sample is supplied, sample loading, the power source for electrophoresis is turned on again to actuate the pulse counter of the signal processing device, in order to resume the measurement and the base sequence decoding. The analysis is completed after a predetermined or fixed analysis period, for example five hours, after the final sample loading.

In the embodiment of Fig. 2, a system was provided wherein the microcomputer 50 is controlled by the computer 33. However, it is also possible to run a microcomputer program with a specially designed control panel and therefore eliminate the computer 33, and the present embodiment all as described above, includes such a modification. Further, the signal processing circuit 32 has been described to use a pulse counter. However, it is also possible to use a circuit including an A/D converter for measuring the intensity of signals. Instead of the power relay, a dedicated interface circuit may be used to provide the same function of controlling the dc power source.

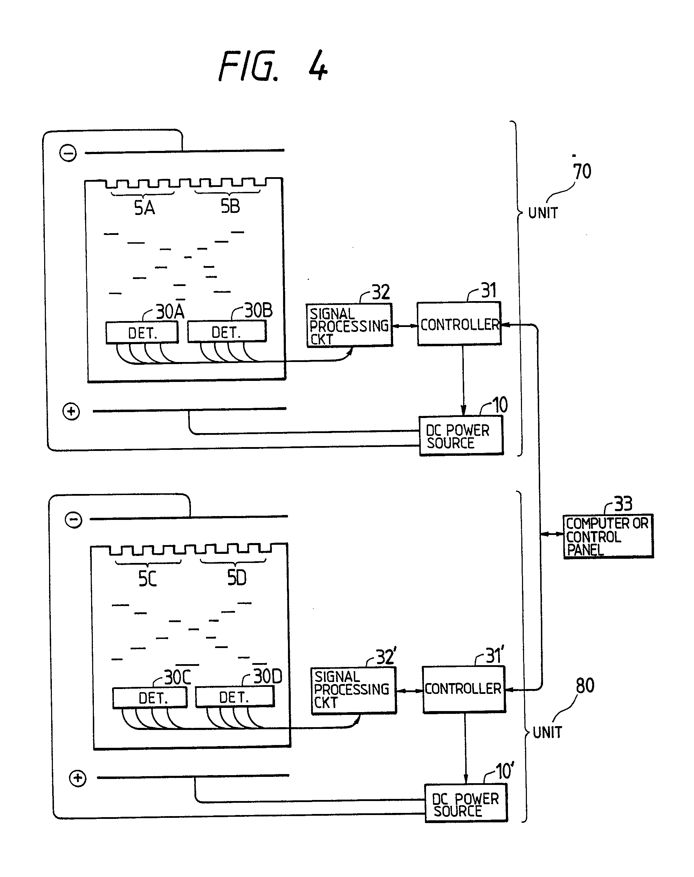

Fig. 4 illustrates the apparatus of Figs. 1 and 2 and the process of Fig. 3 being combined with like apparatus, like processes to provide a larger unit. In this case, the two units of Figs. 1 and 2 are controlled by a single computer 33. Namely, controllers 31 and 31' of the units are independently controlled by the computer 33, and the sample can be added to the unit 80 while the unit 70 is under the condition of being measured.

In the foregoing embodiments, the controller 31 is constituted by a microcomputer. However, the present invention can also be put into practice by using the computer 33 directly control a specially designed circuit which is made up of a combination of a switch for the power source relay and a circuit for controlling the computer. That is, the controller, separate from the computer 33, may be eliminated and the computer 33 provide the function of such controller.

According to the present invention, a sample can be added to the apparatus while it is analyzing another sample, so that a plurality of sets of samples can be continuously analyzed. This makes it possible to continuously analyze a sample prepared at any time. As noted above, the voltage for the apparatus is very high, and accordingly it is necessary to turn off the power supplied for the electrophoresis whenever a new sample is added, for safety reasons.

While preferred embodiments along with variations and modifications have been set forth for disclosing the best mode and important details, further embodiments, variations and modifications are contemplated according to the broader aspects of the present invention, all as set forth in the spirit and scope of the following claims.

## Claims

1. A method for determining the base sequence of a nucleic acid in common apparatus by continuously analyzing samples prepared at any time, comprising the steps of:
labeling a first sample of nucleic acid with a substance that will emit radiation rays;
separating the first sample of labeled nucleic acid into fragments to form a first sample set;
separately contacting the labeling fragments with a first end of a first portion of electrophoretic gel, with the fragments being spaced apart from each other along a contacting direction;
applying an electric voltage from said first end to the opposite end of the electrophoretic gel with a polarity for thereby moving the contacting fragments of labeled nucleic acid from said first end towards the opposite end along respectively separate paths perpendicular to said contacting direction at a rate substantially inversely proportional to the logarithm of the molecular weight of the fragments;
during said step of applying, detecting the rays emitted from said fragments, respectively, on the path of movement for separately detecting the fragments of nucleic acids and producing corresponding electronic detector signals;
storing information corresponding to said signals;
simultaneously interrupting said applying electric voltage across said electrophoretic gel and holding said detecting;
thereafter contacting a second different sample set of fragmented, labeled nucleic acid, with fragments spaced from each other along a contacting direction at a separate second portion of electrophoretic gel;
thereafter repeating said step of applying an electric voltage for said first and second portions of electrophoretic gel;
simultaneously with said repeating step of applying electric voltage, detecting separately the emitted rays from the fragments of the first sample set and producing correlated electrical detector signals, processing the detector signals from the first sample set together with the signals stored for the first set, separately detecting the emitting rays from the fragments of the second set and producing correlated electrical detector signals, and processing the signals from the second set for determining the base sequence of the nucleic acid of the second set for an analyzing period different from and overlapping the analyzing period for determining the base sequence of the nucleic acid of the first set of fragmented samples; and
repeating said steps of detecting and processing separately for respective analyzing periods of the first and second sets.

2. The method according to claim 1, including decoding the DNA sequence of bases of the nucleic acid based on said processing separately for the first and second sample sets.

3. The method according to claim 1, wherein said step of labeling includes radioisotope labeling with a radioactive substance that will emit Beta rays and said step of detecting detects Beta rays.

4. The method according to claim 1, wherein said step of labeling includes labeling with a fluorescent material that will emit light, and said step of detecting detects light.

5. The method according to claim 1, wherein said step of applying electrical voltage applies the voltage so that the negative electrode is at the first end portion so that the fragments travel from the negative electrode to the positive electrode.

6. The method according to claim 1, including a real time step of decoding including removing a decoding program from memory and executing the decoding program with respect to the processed signals with a microcomputer between said steps of detecting automatically during the analyzing period.

7. The method according to claim 6, wherein said steps of decoding of each of the sample sets is performed with the same decoding program from memory and the same microcomputer, at corresponding different times.

8. The method according to claim 7, wherein said detecting is performed with an analog to digital converter, and said step of decoding includes a digital to analog converter.

9. The method according to claim 1 or 7, wherein said step of separating includes chemically fragmenting the nucleic acid into at least four kinds of bases, namely adenine, guanine, cytosine and thymine.

10. The method according to claim 1, 7 or 9, wherein said step of contacting for said first sample set and said step of contacting said second samle set each contact the same sheet of electrophoretic gel at different spaced apart portions thereof.

11. The method according to claim 1, wherein said step of processing includes counting pulses from said step of detecting with a pulse counter.

12. The method according to claim 1, 9 or 10, including providing common equipment that includes a single controller (31), a single DC power source (10), and single processing circuit (33); and wherein said steps of detecting are respectively performed for different sample sets with different detectors (30A, 30B) feeding signals to a common signal processing circuit (32).

13. The method according to claim 1 or 12, including automatically monitoring if an additional sample is to be added, if no additional sample then performing said steps of detecting and processing for a fixed period of time prior to again monitoring if an additional sample is to be added, and if an additional sample is to be added then automatically performing said step of interrupting and holding until completion of the new sample addition.

14. An apparatus for determining the base sequence of a nucleic acid separated into fragments and for continuously analyzing samples that are prepared at any time, comprising:
electrophoretic gel (2) for separately receiving the separated fragments of first and second sample sets of nucleic acid that has been labeled with a ray emitting substance;
power means (10) having opposed electrodes for applying a voltage to said electrophoretic gel for thereby moving fragments respectively at a rate substantially inversely proportional to the molecular weight of the fragments along a path of movement from one electrode to the other electrode;
detector means (30A, 30B) provided on the path of move ment of said fragments of the nucleic acid for separately and repeatedly detecting the rays emitted from said fragments and, thereby detecting the fragments of the nucleic acid;
signal processing means (32) for processing signals from said detector means to provide correlated indications of the fragments;
controller means (31) for simultaneously interrupting said power means (10) and one of said detector means (30A, 30B) and processing means (33); said controller means (31) controlling said signal processing means (33) to store the signals of the first sample set for at least such time as needed to introduce a second sample set;
said controller means (31) thereafter in response to applying a second set, simultaneously activating said power means (10), said detector means (30A, 30B) and said signal processing means (32) for both sample sets; and
said detector means (30A, 30B) and signal processing means separately detecting and processing, respectively, the different sample sets.

15. The apparatus according to claim 14, wherein said detector means (30A, 30B) detects beta rays.

16. The apparatus according to claim 14, wherein said detector means (30A, 30B) detects light emitted from fluorescent labeling material.

17. The apparatus according to claim 14, including a plurality of wells (5A, 5B) contacting the nucleic acid with the electrophoretic gel (2) and arranged in sets for the separate sample sets, with each set including one well (5A, 5B) for each fragment, with the sets of wells being spaced apart from each other along the one end of the electrophoretic gel (2) and in a direction generally perpendicular to said path of movement; said power means (10) including a negative electrode extending along said one end for the entire length of said sets, and a opposite electrode substantially coextensive at the opposite end of the electrophoretic gel (2).

18. The apparatus of claim 14 or 17, further including means (41A, 41B) for separately and repeatedly decoding the sequence of bases of each nucleic acid based upon the signals from said signal processing means (32) on a real time basis with said detector means, respectively.

19. The apparatus of claim 14 or 17, including two separate electrophoretic gels (2), each having separate sets of wells (5A, 5B; 5C, 5D), detector means, and power means electrodes; said power means (10, 10') being common to said electrophoretic gels (2); and further including a computer (33) and computer control program common to said detector means (30A, 30B; 30C, 30D), power means (10, 10'), processing means (32, 32') and controller means (31, 31').

20. An apparatus for determining the base sequence of a nucleic acid separated into fragments, comprising:

electrophoretic gel (2) for separately receiving the separated fragments of nucleic acid that has been labeled with a ray emitting substance;

power means (10) having opposed electrodes for applying a voltage to said electrophoretic gel for thereby moving fragments respectively at a rate substantially inversely proportional to the molecular weight of the fragments along a path of movement from one electrode to the other electrode;

detector means (30A, 30B) provided on the path of movement of said fragments of the nucleic acid for separately and repeatedly detecting the rays emitted from said fragments and, thereby detecting the fragments of the nucleic acid;

signal processing means (32) for processing signals from said detector means to provide correlated indications of the fragments;

decoding means (41A, 41B) for separately and repeatedly decoding the sequence of bases of each nucleic acid based upon the signals from said signal processing means on a real time basis with said detector means, respectively; and

controller means (31) for said power means (10), said decoding means (41A, 41B), said processing means (32) and said detector means (30A, 30B), for repeating their operation automatically over an analyzing period of time and to store data.

21. A method for determining the base sequence of a nucleic acid, comprising the steps of:

labeling a sample of nucleic acid with a substance that will emit radiation rays;

separating the sample of labeled nucleic acid into fragments to form a sample set;

separately contacting the labeled fragments with a first end of a first portion of electrophoretic gel, with the fragments being spaced apart from each other along a contacting direction;

applying an electric voltage from said first end to the opposite end of the electrophoretic gel with a polarity for thereby moving the contacting fragments of labeled nucleic acid from said first end towards the opposite end along respectively separate paths perpendicular to said contacting direction at a rate substantially inversely proportional to the logarithm of the molecular weight of the fragments;

during said step of applying, detecting the rays emitted from said fragments, respectively, on the path of movement for separately detecting the fragments of nucleic acids and producing corresponding electronic detector signals;

storing information corresponding to said signals;

simultaneously with said step of applying electric voltage, detecting separately the emitted rays from the fragments of the sample set and producing correlated electrical additional detector signals, processing the additional detector signals from the sample set together with the signals stored;

repeating said steps of detecting and processing automatically for an analyzing period of the set; and

real time decoding the processed signals with a microcomputer between said steps of detecting to determine the base DNA sequence of the sample on a real time basis during the analyzing period.

22. An apparatus for determining the base sequence of a nucleic acid separated into fragments, comprising: an electrophoretic gel (2) for receiving the fragments; power source means (10) for applying a voltage to said electrophoretic gel (2) for thereby moving said fragments of nucleic acid along a path in said electrophoretic gel (2) depending upon their molecular weights;

detector means (30A, 30B) provided on the path of said fragments of nucleic acid for detecting said fragments of nucleic acid;

signal processing means (32) for processing signals from said detector means (30A, 30B) and

controller means (31) for interrupting said power source means and controlling said signal processing means.

23. The apparatus according to claim 22, further including memory means (52) for storing information related to said signals during interruption of said power source (10), and said controller means (32) further processing said stored information together with new signals from said detector means (30A, 30B) when said power means again applies voltage after previously being interrupted.

24. The apparatus according to claim 22 or 23, wherein said controller means (32) decodes the sequence of bases of the nucleic acid based upon the signals on a real time basis with said detector means.

25. A method for determining the base sequence of nucleic acid, comprising the steps of:

labeling a sample of nucleic acid with a substance that will emit radiation rays;

separating the sample of labeled nucleic acid into fragments to form a sample set;

separately contacting the labeling fragments with a first end portion of electrophoretic gel, with the fragments being spaced apart from each other along a contacting direction;

applying an electric voltage from said first end to the opposite end of the electrophoretic gel with a polarity for thereby moving the contracting fragments of labeled nucleic acid from said first end towards the opposite end along respectively separate paths perpendicular to said contacting direction at a rate substantially inversely proportional to logarithm of the molecular weight of the fragment;

during said step of applying, detecting the rays emitted from said fragments, respectively, on the path of movement for separately detecting the fragments of nucleic acids and for producing corresponding electronic detector signals;
continuously repeating said step of detecting for a fixed analyzing period; and
removing a decoding program from memory and executing the decoding program by repeatedly decoding the signals on a real time basis with said step of detecting.

26. Apparatus for determining the base sequence of a nucleic acid separated into fragments, comprising:
electrophoretic gel (2) for separately receiving the separated fragments of a set of nucleic acid that has been labeled with a ray emitting substance;
power means (10) having opposed electrodes for applying a voltage to said electrophoretic gel (2) for thereby moving fragments respectively at a rate substantially inversely proportional to the molecular weight of the fragments along a path of movement from one electrode to the other electrode;
detector means (30A, 30B) provided on the path of movement of said fragments of the nucleic acid for separately and repeatedly detecting the rays emitted from said fragments, and thereby detecting the fragments of the nucleic acid and producing corresponding electrical signals;
signal processing means (32) for processing the electrical signals from said detector means to provide correlated indications of the fragments; and
controller means (31) for storing a decoding program and executing the decoding program on a real time basis with said detector means by repeatedly decoding the correlated indications of the fragments from said signal processing means.

# FIG. 1

# FIG. 2

# FIG. 3

```
                    ┌─────────────────────┐ ─100
                    │   SAMPLE LOADING    │
                    └─────────────────────┘
                              │
                    ┌─────────────────────┐ ─110
                    │ POWER ON, COUNTER ON│
                    └─────────────────────┘
                              │
                              │
                    ┌─────────────────────┐ ─120
                    │      MEASURE        │
                    └─────────────────────┘
                              │
                           ◇─130
                        NEW SAMPLE        YES ──────┐
                            ?                        │
                           ◇                         ▼
                            │ NO              ┌──────────────────────┐ ─180
                            │                 │ POWER OFF, COUNTER OFF│
                            │                 └──────────────────────┘
                    ┌─────────────────────┐ ─140         │
                    │TRANSFER DATA TO CONTROLLER│  ┌──────────────────────┐ ─190
                    └─────────────────────┘        │   SAMPLE LOADING     │
                              │                     └──────────────────────┘
                    ┌─────────────────────┐ ─150         │
                    │      DECODING       │        ┌──────────────────────┐ ─200
                    └─────────────────────┘        │ POWER ON, COUNTER ON │
                              │                     └──────────────────────┘
                              │◄────────────────────────────┘
                           ◇─160
                   NO   ANALYSIS
                  ◄──   FINISHED
                            ?
                           ◇
                            │ YES
                    ┌─────────────────────┐ ─170
                    │        END          │
                    └─────────────────────┘
```

# FIG. 4

# FIG. 5 PRIOR ART